# EUROPÄISCHE PATENTSCHRIFT

(11) **EP 1 309 538 B1**
(45) Veröffentlichungstag und Bekanntmachung des Hinweises auf die Patenterteilung: **11.01.2006**
(21) Anmeldenummer: 01960238.2
(22) Anmeldetag: 23.05.2001
(51) Int. Cl.: C07C 51/367

(54) **VERFAHREN ZUR HERSTELLUNG VON TRIFLUORETHOXYSUBSTITUIERTEN BENZOESÄUREN**
METHOD FOR THE PRODUCTION OF TRIFLUOROETHOXY-SUBSTITUTED BENZOIC ACIDS
PROCEDE DE PRODUCTION D'ACIDES BENZOIQUES SUBSTITUES PAR TRIFLUOROETHOXY

(30) Priorität: 26.05.2000 DE 10025700
(43) Veröffentlichungstag der Anmeldung: 14.05.2003
(73) Patentinhaber: Merck Patent GmbH, 64293 Darmstadt (DE)
(72) Erfinder: FABIAN, Kai, 69259 Wilhelmsfeld (DE); ENKE, Steffen, 68535 Neckarhausen (DE); TILLY, Herbert, 64319 Pfungstadt (DE)
(86) Internationale Anmeldenummer: PCT/EP2001/005923
(87) Internationale Veröffentlichungsnummer: WO 2001/090062

(56) Entgegenhaltungen:
- WO-A-98/47853
- DE-A- 3 931 954
- J. WROBEL ET AL.: "Syntheses of Tolrestat Analogues Containing Additional Substituents in the Ring and Their Evaluation as Aldose Reductase Inhibitors. Identification of Potent, Orally Active 2-Fluoro Derivatives" J. MED. CHEM., Bd. 34, Nr. 8, 1991, Seiten 2504-2520, XP000567039

## Beschreibung

Die Erfindung betrifft ein Verfahren zur Herstellung von trifluorethoxysubstituierten Benzoesäuren der Formel I 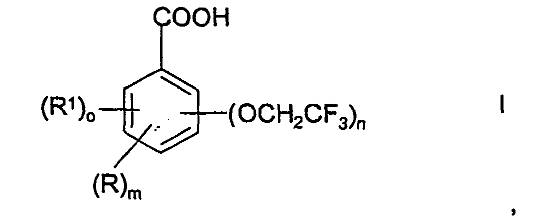
worin
- R, R¹: jeweils unabhängig voneinander A, OA, COOH, COOA, SA, CF₃, OCF₃, CN, NO₂, Hal, -(CH₂)ₚ-Hal, -O-(CH₂)ₚ-Hal, -S-(CH₂)ₚ-Hal, Ar, -(CH₂)ₚ-Ar, OAr, O(CO)Ar, NH₂, NHA, NA₂, CONH₂, CONHA oder CONA₂,
- A: einen Alkylrest mit 1 bis 4 C-Atomen,
- Ar: unsubstituiertes oder ein-, zwei- oder dreifach durch A, OH, OA, CF₃, OCF₃, CN oder NO₂ substituiertes Phenyl oder Naphthyl,
- Hal: F, Cl, Br oder I,
- n: 1, 2 oder 3,
- m, o: jeweils unabhängig voneinander 0, 1 oder 2,
- p: 1 oder 2
bedeutet,
durch Umsetzung einer Verbindung der Formel II 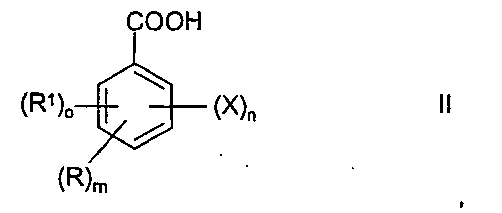
worin
- X: Cl, Br oder I bedeutet und

R, R¹, A, Ar, Hal, n, m, o und p die in Formel I angegebene Bedeutung haben,
wobei für n > 1 X gleich oder verschieden sein kann, dadurch gekennzeichnet,
dass man Kalium-tert.-butanolat und ein Tris-(polyoxaalkyl)-amin als Phasentransfer-Katalysator in THF vorlegt, gegebenenfalls den Phasentransfer-Katalysator oder die Base, sofern sie nicht zusammen eingetragen wurden, anschließend zugibt, 2,2,2-Trifluorethanol zutropft und diesem Reaktionsgemisch nacheinander oder zeitgleich in beliebiger Reihenfolge Kupfer(I)-bromid und die entsprechende halogenierte Benzoesäure der Formel II zufügt,
und anschließend sauer aufarbeitet.

Trifluorethoxysubstituierte Benzoesäurederivate der Formel I sind nützliche Synthesebausteine, z.B. für die Herstellung von Arzneimitteln.
Insbesondere die Verbindung 2,5-Bis(2,2,2-trifluorethoxy)benzoesäure ist ein wichtiges Zwischenprodukt bei der Synthese des Wirkstoffs Flecainide Acetat, einem Antiarrhythmicum, bekannt aus US 3,900,481.

Ein bekanntes Herstellungsverfahren von trifluorethoxysubstituierten Benzoesäurederivaten der Formel I wird in Banitt et al, J. Med Chem. 1975, 18, 1130 beschrieben. Hydroxysubstituierte Benzoesäuren oder Benzoesäureester werden in einer klassischen nucleophilen Substitution mit 2,2,2-Trifluorethyl-trifluormethansulfonat umgesetzt.

GB 2045760 beschreibt ein Verfahren zur Herstellung von 2,5-Bis(2,2,2-trifluorethoxy)benzoesäure, indem zunächst Hydrochinon oder 1,4-Dibrombenzol mit 2,2,2-Trifluorethyl-trifluormethansulfonat oder Trifluorethanolat zu 1,4-Di-(trifluorethoxy)benzot umgesetzt wird, die Acetylgruppe in o-Position zu einem Trifluorethoxy-Substituenten durch Acetylierung in Gegenwart einer Lewis-Säure eingeführt und nachfolgend zur gewünschten Benzoesäure oxidiert wird.

Die bisher beschriebenen Verfahren benötigen entweder teuere und kommerziell eingeschränkt verfügbare Rohstoffe oder stützen sich auf eine mehrstufige Synthesesequenz, die für eine großtechnische Anwendung unpraktikabel ist.

Diese Nachteile werden durch die Einstufen-Synthese der WO 98/47853 behoben. In WO 98/47853 werden halogenierte Benzoesäuren oder deren Salze mit 2,2,2-Trifluorethanol in Gegenwart einer starken Base und einem

Kupfer-enthaltenden Material umgesetzt und gegebenenfalls sauer aufgearbeitet. Dieser Reaktionstyp entspricht einer Ullmann Reaktion. Weiterhin wird die Verwendung eines aprotischen Lösungsmittels, insbesondere der Lösungsmittel N,N-Dimethylformamid, N-Methylpyrrolidon, N,N-Dimethylacetamid, Pyridin, Collidin, Dimethylsulfoxid, Hexamethylphosphoramid oder Mischungen davon, als vorteilhaft angesehen. Trifluorethanol kann jedoch auch selbst als Lösungsmittel verwendet werden. Für das beschriebene Verfahren geeignete starke Basen sind Natrium, NaH, NaNH₂, Natrium- oder Kaliumalkoholate, NaOH, KOH, am Stickstoff substituierte Amidine, Guanidine oder Tetraalkylammoniumhydroxide. Als eine besonders bevorzugte Base wird Natriumhydrid offenbart.
Die Reaktionstemperatur beträgt, aufgrund von DMF als bevorzugtes Lösungsmittel, vorzugsweise 110-115°C.
Nach saurer Hydrolyse fällt das Reaktionsprodukt zusammen mit den entstandenen Kupfersalzen und Nebenprodukten aus und wird abfiltriert. Der Rückstand wird zunächst in 5%iger KOH aufgenommen und die Lösung über Celit filtriert. Die verbleibende alkalische Lösung wird mehrmals mit beispielsweise Dichlormethan extrahiert und anschließend das Produkt aus der alkalischen Lösung mit Salzsäure gefällt und umkristallisiert.

Das System NaH mit den oben aufgelisteten Lösungsmitteln, insbesondere DMF, besitzt jedoch für eine großtechnische Synthese ein hohes Sicherheitsrisiko, da sich dieses System unkontrollierbar exotherm zersetzen kann (Lit.: Gordon DeWall, C&EN, 1982, Sept. 13, S. 42-43). Zusätzlich entsteht bei der Hydrolyse des verbleibenden Natriumhydrids immer Wasserstoff, der wiederum ein zweites Sicherheitsrisiko birgt. Auch der Wechsel auf andere Basen, z.B. der Einsatz von Natrium- oder Kalium-tert-butanolat, in Kombination mit den aus WO 98/47853 offenbarten Lösungsmitteln, insbesondere DMF, führt nicht zu einer hinsichtlich der Sicherheitsvorgaben für großtechnische Synthesen zufriedenstellenden Kontrollierbarkeit der Reaktion. Das aus WO 98/47853 bekannte Verfahren, wie zuvor beschrieben, eignet sich daher nur für Reaktionen im Labormaßstab.

Der Erfindung lag daher die Aufgabe zugrunde, ein verbessertes Verfahren zur Herstellung von trifluorethoxysubstituierten Benzoesäurederivaten der Formel I zu entwickeln. Das verbesserte Verfahren sollte sich insbesondere für eine großtechnische Synthese eignen.

Das erfindungsgemäße Verfahren stellt eine Auswahlerfindung zu dem in WO 98/47853 beschriebenen Verfahren dar.

Überraschenderweise wurde gefunden, dass die Reaktion der halogenierten Benzoesäurederivate der Formel II, wie zuvor beschrieben, mit Trifluorethanol in Gegenwart einer Base und einem Kupfersalz bei Verwendung des Lösungsmittels THF in Gegenwart der Basen Natrium, NaH, NaNH₂, Na- oder K-Alkoholat, NaOH oder KOH, insbesondere in Gegenwart von Kalium-tert-butanolat, zu einer vom sicherheitstechnischen Standpunkt aus sehr gut kontrollierbaren Reaktion führt.
Die Umsetzung der Edukte zu den trifluorethoxysubstituierten Benzoesäurederivaten der Formel I gelingt in guten bis sehr guten Ausbeuten bei einer zu WO 98/47853 vergleichsweise geringen Reaktionstemperatur von ca. 70°C.
Weiterhin erleichtert sich durch die Verwendung des Lösungsmittels Tetrahydrofuran in Anwesenheit von verdünnter Salzsäure die Abtrennung der aus der Reaktion entstehenden Kupfersalze. Die Aufarbeitung nach dem erfindungsgemäßen Verfahren benötigt im Vergleich zu WO 98/47853 eine geringere Anzahl an Arbeitsschritten, da das Produkt in THF gelöst bleibt, während die Kupfersalze und Nebenprodukte in überwiegender Menge in der wäßrigen Phase verbleiben. Insbesondere entfällt die Nachextraktion mit einem halogenierten Lösungsmittel, wie für das Verfahren der WO 98/47853 beschrieben.

Bedingt durch die im Vergleich zu dem bevorzugten System der WO 98/47853 niedrigere Reaktionstemperatur, wie zuvor beschrieben, wird die Bildung von Nebenprodukten unterdrückt, so dass Produkte mit hoher Reinheit entstehen.

Gegenstand der Erfindung ist daher ein Verfahren zur Herstellung von trifluorethoxysubstituierten Benzoesäuren der Formel I 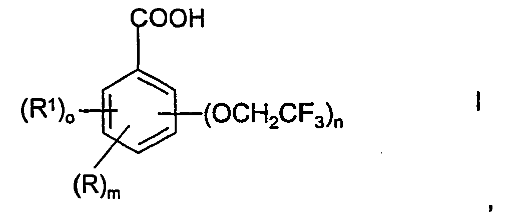
worin
- R, R¹: jeweils unabhängig voneinander A, OA, COOH, COOA, SA, CF₃, OCF₃, CN, NO₂, Hal, -(CH₂)ₚ-Hal, -O-(CH₂)ₚ-Hal, -S-(CH₂)ₚ-Hal, Ar, -(CH₂)ₚ-Ar, OAr, O(CO)Ar, NH₂, NHA, NA₂, CONH₂, CONHA oder CONA₂,
- A: einen Alkylrest mit 1 bis 4 C-Atomen,
- Ar: unsubstituiertes oder ein-, zwei- oder dreifach durch A, OH, OA, CF₃, OCF₃, CN oder NO₂ substituiertes Phenyl oder Naphthyl,
- Hal: F, Cl, Br oder I,
- n: 1, 2 oder 3,
- m, o: jeweils unabhängig voneinander 0, 1 oder 2,
- p: 1 oder 2
bedeutet,
durch Umsetzung einer Verbindung der Formel II 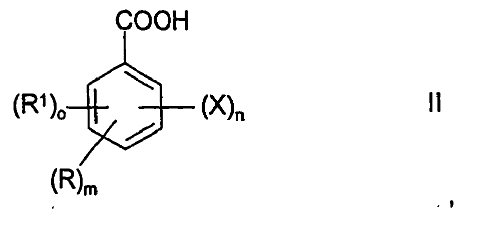 worin
- X: Cl, Br oder I bedeutet und

R, R¹, A, Ar, Hal, n, m, o und p die in Formel I angegebene Bedeutung haben,
wobei für n>1 X gleich oder verschieden sein kann,
dadurch gekennzeichnet,
dass man Kalium-tert.-butanolat und ein Tris-(polyoxaalkyl)-amin als Phasentransfer-Katalysator in THF vorlegt, gegebenenfalls den Phasentransfer-Katalysator oder die Base, sofern sie nicht zusammen eingetragen wurden, anschließend zugibt, 2,2,2-Trifluorethanol zutropft und diesem Reaktionsgemisch nacheinander oder zeitgleich in beliebiger Reihenfolge Kupfer(I)-bromid und die entsprechende halogenierte Benzoesäure der Formel II zufügt,
und anschließend sauer aufarbeitet.

Das erfindungsgemäße Verfahren, wie zuvor beschrieben, eignet sich in besonderer Weise für großtechnische Synthesen, d.h. vorzugsweise für die Herstellung von Produkten im Bereich von 1 kg bis 500 kg.

In den vorstehenden Formeln ist A Alkyl und hat 1 bis 4, vorzugsweise 1, 2 oder 3 C-Atome. Alkyl bedeutet vorzugsweise Methyl, weiterhin Ethyl, Propyl, Isopropyl, Butyl, Isobutyl, sec.-Butyl oder tert.-Butyl. Besonders bevorzugt is A Methyl.

Ar bedeutet vorzugsweise unsubstituiertes Phenyl oder Naphthyl, weiterhin vorzugsweise durch A, OH, OA, CF₃, OCF₃, CN oder NO₂ mono-, di- oder trisubstituiertes Phenyl. A hat eine der zuvor angeführten bevorzugten Bedeutungen. Besonders bevorzugt ist Ar unsubstituiertes Phenyl.

-(CH₂)ₚ-Ar mit p = 1 oder 2, bedeutet auch arylalkyl und ist bevorzugt Benzyl, Phenylethyl oder Naphthylmethyl, besonders bevorzugt Benzyl.

O(CO)Ar bedeutet bevorzugt Benzoyl.

Hal bedeutet bevorzugt F oder Cl.

X bedeutet bevorzugt Cl oder Br.

R oder R¹ bedeuten jeweils unabhängig voneinander A, OA, COOH, COOA, SA, CF₃, OCF₃, CN, NO₂, Hal, -(CH₂)ₚ-Hal, -O-(CH₂)ₚ-Hal, -S-(CH₂)ₚ-Hal, Ar, -(CH₂)ₚ-Ar, OAr, O(CO)Ar, NH₂, NHA, NA₂, CONH₂, CONHA oder CONA₂. Besonders bevorzugt für R und R¹, jeweils unabhängig voneinander, ist A, OA oder NH₂. Ganz besonders bevorzugt sind R und R¹ H.

p ist bevorzugt 1.
n ist bevorzugt 1 oder 2, besonders bevorzugt 2.
m ist bevorzugt 0 oder 1, besonders bevorzugt 0.
o ist bevorzugt 0.

Das erfindungsgemäße Verfahren eignet sich in besonders bevorzugter Weise zur Synthese von 2,5-Bis(2,2,2-trifluorethoxy)benzoesäure.

Die halogenierten Benzoesäurederivate der Formel II sind kommerziell erhältlich oder können nach an sich bekannten Methoden wie z.B. in Houben-Weyl, Methoden der Organ. Chemie beschrieben, hergestellt werden. Bevorzugtes Edukt für die Synthese von 2,5-Bis(2,2,2-trifluorethoxy)benzoesäure ist 5-Brom-2-chlorbenzoesäure. Weiterhin ist auch der Einsatz eines Salzes der Benzoesäurederivate der Formel II, beispielsweise ein Natrium- oder Kaliumbenzoat der Formel II, als Edukt in dem erfindungsgemäßen Verfahren geeignet.

Phasentransfer-Katalysatoren aus der Klasse der Tris(polyoxaalkyl)amine sind für Ullmann bzw. Ullmann-analoge Reaktionen aus der Literatur bekannt (Lit.: G. Soula, J. Org. Chem. 1985, 50, 3717-3721 oder Rewcastle et al, J. Med. Chem. 1989, 32, 793-799). Für das erfindungsgemäße Verfahren besonders geeignet sind die Phasentransfer-Katalysatoren Tris[2-(2-methoxy)ethoxy]-ethylamin (TDA-1) oder Tris[2-(2-ethoxy)ethoxy]ethylamin (TDA-2), besonders bevorzugt wird TDA-1 verwendet.

Gegenstand der Erfindung ist ein Verfahren, wie zuvor beschrieben, dadurch gekennzeichnet, dass der Phasentransfer-Katalysator ein Tris-(polyoxaalkyl)-amin ist. In einer besonders bevorzugten Ausführungsform wird das erfindungsgemäße Verfahren in Anwesenheit von TDA-1 durchgeführt.

Für das erfindungsgemäße Verfahren, wie zuvor beschrieben, eignen sich die Basen Natrium, NaH, NaNH₂, Na- oder K-Alkoholat, NaOH oder KOH. Bevorzugt eignen sich Na- oder K-Alkoholate, beispielsweise Natrium- oder Kaliummethanolat, Natrium- oder Kaliumethanolat oder Natrium- oder Kalium-tert.-butanolat. Besonders bevorzugt wird im erfindungsgemäßen Verfahren Kalium-tert.-butanolat verwendet. Auf die Verwendung der Base kann teilweise oder auch ganz verzichtet werden, wenn anstelle des Trifluorethanols ein entsprechendes Trifluorethanolat eingesetzt wird. Für das erfindungsgemäße Verfahren ist insbesondere Natrium- oder Kaliumtrifluorethanolat geeignet.

Gegenstand der Erfindung ist ein Verfahren, wie zuvor beschrieben, dadurch gekennzeichnet, dass die Base aus der Gruppe Natrium, NaH, NaNH₂, Na- oder K-Alkoholat, NaOH oder KOH ausgewählt wird.

Allgemein werden unter den Kupfersalzen Kupfer(I)salze verstanden, beispielsweise Cu(I)acetat, Cu(I)Br, Cu(I)Cl, Cu(I)I, Cu(I)oxid, oder Cu(I)rhodanid. Erfindungsgemäß eignen sich insbesondere die Kupfersalze Cu(I)Cl, Cu(I)Br oder Cu(I)I, wobei der Einsatz von Cu(I)Br besonders bevorzugt ist.

Gegenstand der Erfindung ist ebenfalls ein Verfahren, wie zuvor beschrieben, dadurch gekennzeichnet, dass als Kupfersalz Kupfer(I)iodid oder Kupfer(I)bromid verwendet wird.

Die erfindungsgemäße Umsetzung, wie zuvor beschrieben, wird bevorzugt bei Temperaturen zwischen 10 und 80°, besonders bevorzugt bei Temperaturen zwischen 50 und 70°C durchgeführt. Ganz besonders bevorzugt wird die Reaktion bei Siedetemperatur des Tetrahydrofurans durchgeführt.
Die Temperaturführung des erfindungsgemäßen Verfahrens wird so gewählt, dass die Edukte zunächst bei Temperaturen kleiner 35°C, bevorzugt zwischen 10° und 30°C, besonders bevorzugt bei 20°C gemischt werden und die Temperatur danach auf die eigentliche Reaktionstemperatur zwischen 50° und 80°, insbesondere auf die Siedetemperatur des Tetrahydrofurans, erhöht wird.

Gegenstand der Erfindung ist auch ein Verfahren, wie beschrieben,
dadurch gekennzeichnet, dass man die Reaktion bei Temperaturen zwischen 10° und 80°C durchführt.
Gegenstand der Erfindung ist ein Verfahren, wie zuvor beschrieben, wobei die Edukte bei einer Temperatur kleiner 35°C gemischt werden und die Temperatur des Reaktionsgemisches auf eine Reaktionstemperatur zwischen 50° und 80°C erhöht wird.

Unter dem Begriff Edukte hinsichtlich der Beschreibung der Temperaturführung, werden alle Komponenten der Reaktion zusammengefaßt. Unter den Begriff Edukte fällt (im Zusammenhang mit der Temperaturführung) THF, die Base, der Phasentransfer-Katalysator, Trifluorethanol, das Kupfersalz und eine halogenierte Benzoesäure der Formel II.

Gegenstand der Erfindung ist auch ein Verfahren, wie beschrieben, dadurch gekennzeichnet, dass man das Reaktionsgemisch sauer aufarbeitet. Die saure Hydrolyse wird bevorzugt mit einer Säure, ausgewählt aus einer Gruppe von Säuren, zu der organische Säuren, bevorzugt Ameisensäure, Essigsäure oder Propionsäure oder auch anorganische Säuren, bevorzugt Schwefelsäure, Salpetersäure, Halogenwasserstoffsäuren wie Chlorwasserstoffsäure oder Bromwasserstoffsäure oder Phosphorsäuren wie Orthophosphorsäure gehören, durchgeführt. Besonders bevorzugt eingesetzt wird Chlorwasserstoffsäure.

Für das erfindungsgemäße Verfahren geeignete molare Verhältnisse ausgewählter Komponenten zueinander wird im folgenden beschrieben.

Verwendet wird mindestens 1 mol 2,2,2-Trifluorethanol pro Halogenatom einer Verbindung der Formel II, wobei bevorzugt mit einem Überschuß an Trifluorethanol gearbeitet wird. Besonders bevorzugt werden 3 mol 2,2,2-Trifluorethanol pro Halogenatom einer Verbindung der Formel II eingesetzt.

Verwendet wird mindestens 1 mol 2,2,2-Trifluorethanol pro mol Base.

Das molare Verhältnis von Kupfersalz zu einer Verbindung der Formel II kann 0,01:1 bis 2:1 betragen. Besonders bevorzugt ist ein molares Verhältnis von 1:1.

Das molare Verhältnis von Phasentransfer-Katalysator zu einer Verbindung der Formel II kann 0,01:1 bis 1:1 betragen. Bevorzugt ist ein molares Verhältnis von 0,1:1 bis 0,5:1, ganz besonders bevorzugt ist ein molares Verhältnis von 0,2:1.

Gegenstand der Erfindung ist ferner ein Verfahren zur Herstellung von trifluorethoxysubstituierten Benzoesäurederivaten der Formel I, dadurch gekennzeichnet, dass man die Base und/oder gegebenenfalls den Phasentransfer-Katalysator in THF vorlegt, gegebenenfalls den Phasentransfer-Katalysator oder die Base, sofern sie nicht zusammen eingetragen wurden, anschließend zugibt, 2,2,2-Trifluorethanol zutropft und diesem Reaktionsgemisch nacheinander oder zeitgleich in beliebiger Reihenfolge das Kupfersalz und die entsprechende halogenierte Benzoesäure der Formel II zufügt.
Nach einer Reaktionszeit von einigen Minuten bis mehreren Stunden, wird die Reaktionsmischung gekühlt und die Säure, wie zuvor beschrieben, zugesetzt. Die Aufarbeitung der Reaktionsmischung erfolgt nach Techniken, die dem Fachmann bekannt sind oder wie in den folgenden Beispielen erläutert.

Auch ohne weitere Ausführungen wird davon ausgegangen, dass ein Fachmann die obige Beschreibung in weitestem Umfang nutzen kann. Die bevorzugten Ausführungsformen sind deswegen lediglich als beschreibende, keineswegs als in irgendeiner Weise limitierende Offenbarung aufzufassen.

Bei den nachfolgenden Beispielen und auch bei den vorherigen Ausführungen wird die Temperatur in °C angegeben. Der pH-Wert entspricht dem dekadischen Logarithmus der H⁺-lonenkonzentration. Die Reinheit des Produktes wird über eine HPLC-Messung bestimmt.

### Beispiel 1:

Bei Raumtemperatur werden 300 ml THF vorgelegt und unter Rühren 84,7 g Kalium-tert.-butanolat zugegeben. Zu diesem Reaktionsgemisch werden 76,0 g 2,2,2-Trifluorethanol zugetropft, wobei die Temperatur unter 35°C gehalten wird. Nach beendeter Zugabe wird nachgerührt und anschließend 29,6 g 5-Brom-2-chlorbenzoesäure eingetragen. Nach darauffolgender Zugabe von 27,3 g Kupfer(I)bromid wird die Reaktionsmischung zum Rückfluß erhitzt.
Nach 43 Stunden wird die Reaktionsmischung auf 5°C abgekühlt und auf verdünnte Salzsäure von 5°C zulaufen lassen. Die organische Phase wird von der wäßrigen Phase getrennt und das Lösungsmittel abdestilliert, wobei das Produkt ausfällt. Zu dem Rückstand werden 100 ml Wasser zugegeben und danach filtriert. Zur Aufreinigung wird das Rohprodukt in MTB-Ether (Methyl-tert.-butyl-ether) aufgenommen. Ungelöste Anteile werden durch Filtration über neutralem Aluminiumoxid abgetrennt und anschließend das Lösungsmittel entfernt. Nach Umkristallisation aus einem Ethanol-Wasser-Gemisch erhält man die 2,5-Bis(2,2,2-trifluorethoxy)benzoesäure in einer Ausbeute von 45%.
Schmelzpunkt: 120-122°C, Reinheit > 98% (HPLC).

### Beispiel 2:

Analog zu Beispiel 1 werden 1500 ml THF vorgelegt und 40,4 g Katalysatoren Tris[2-(2-methoxy)ethoxy]-ethylamin und anschließend 423,6 g Kalium-tert-butanolat unter Rühren eingetragen. Nach Zugabe von 380,1 g 2,2,2-Trifluorethanol, 148,2 g 5-Brom-2-chlorbenzoesäure und 98,8 g Kupfer(I)-bromid wird wie unter Beispiel 1 beschrieben aufgearbeitet.
Nach Umkristallisation aus einem Ethanol-Wasser-Gemisch erhält man die 2,5-Bis(2,2,2-trifluorethoxy)benzoesäure in einer Ausbeute von 68%. Reinheit > 98% (HPLC).

## Patentansprüche

1. Verfahren zur Herstellung von trifluorethoxysubstituierten Benzoesäuren der Formel I 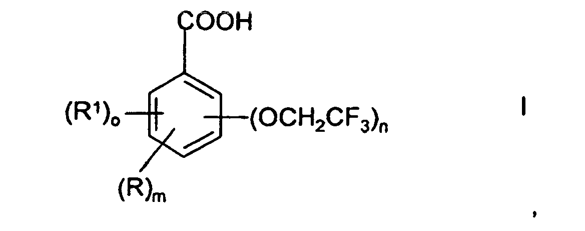
worin
R, R¹ jeweils unabhängig voneinander A, OA, COOH, COOA, SA, CF₃, OCF₃, CN, NO₂, Hal, -(CH₂)ₚ-Hal, -O-(CH₂)ₚ-Hal, -S-(CH₂)ₚ-Hal, Ar, -(CH₂)ₚ-Ar, OAr, O(CO)Ar, NH₂, NHA, NA₂, CONH₂, CONHA oder CONA₂,
A einen Alkylrest mit 1 bis 4 C-Atomen,
Ar unsubstituiertes oder ein-, zwei- oder dreifach durch A, OH, OA, CF₃, OCF₃, CN oder NO₂ substituiertes Phenyl oder Naphthyl,
Hal F, Cl, Br oder I,
n 1, 2 oder 3,
m, o jeweils unabhängig voneinander 0, 1 oder 2,
p 1 oder 2
bedeutet,
durch Umsetzung einer Verbindung der Formel II 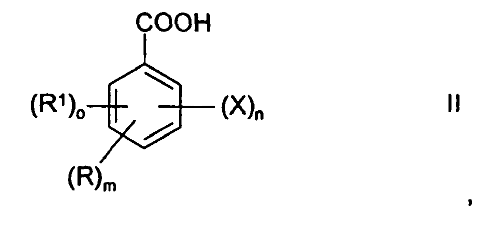
worin
X Cl, Br oder I bedeutet und
R, R¹, A, Ar, Hal, n, m, o und p die in Formel I angegebene Bedeutung haben,
wobei für n>1 X gleich oder verschieden sein kann,
**dadurch gekennzeichnet,**
**dass** man Kalium-tert.-butanolat und ein Tris-(polyoxaalkyl)-amin als Phasentransfer-Katalysator in THF vorlegt, gegebenenfalls den Phasentransfer-Katalysator oder die Base, sofern sie nicht zusammen eingetragen wurden, anschließend zugibt, 2,2,2-Trifluorethanol zutropft und diesem Reaktionsgemisch nacheinander oder zeitgleich in beliebiger Reihenfolge Kupfer(I)-bromid und die entsprechende halogenierte Benzoesäure der Formel II zufügt,
und anschließend sauer aufarbeitet.

2. Verfahren nach Anspruch 1, **dadurch gekennzeichnet, dass** man die Reaktion bei Temperaturen zwischen 10° und 80°C durchführt.

3. Verfahren nach Anspruch 1 oder 2, **dadurch gekennzeichnet, dass** 2,5-Bis(2,2,2-trifluorethoxy)benzoesäure hergestellt wird.

## Claims

1. Process for the preparation of trifluoroethoxy-substituted benzoic acids of the formula I 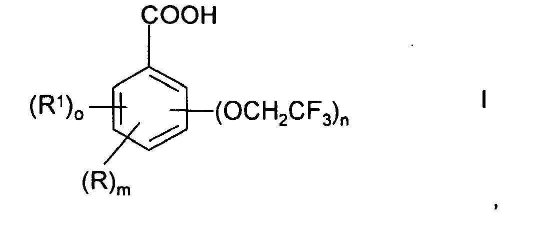
in which
R, R¹ each, independently of one another, denotes A, OA, COOH, COOA, SA, CF₃, OCF₃, CN, NO₂, Hal, -(CH₂)ₚ-Hal, -O-(CH₂)ₚ-Hal, -S-(CH₂)ₚ-Hal, Ar, -(CH₂)ₚ-Ar, OAr, O(CO)Ar, NH₂, NHA, NA₂, CONH₂, CONHA or CONA₂,
A denotes an alkyl radical having 1 to 4 C atoms,
Ar denotes phenyl or naphthyl, each of which is unsubstituted or mono-, di- or trisubstituted by A, OH, OA, CF₃, OCF₃, CN or NO₂,
Hal denotes F, Cl, Br or I,
n denotes 1, 2 or 3,
m, o each, independently of one another, denotes 0, 1 or 2,
p denotes 1 or 2,
by reaction of a compound of the formula II 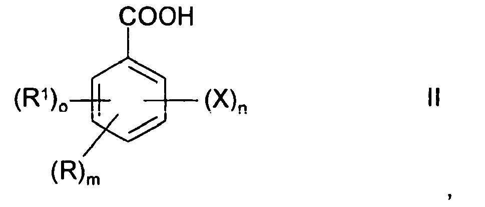
in which
X denotes Cl, Br or I and
R, R¹, A, Ar, Hal, n, m, o and p have the meaning indicated in the formula I, where, for n>1, X may be identical or different,
**characterised in that**
potassium tert-butoxide and a tris(polyoxaalkyl)amine as phase-transfer catalyst are presented in THF, the phase-transfer catalyst, where used, or the base, if they were not introduced together, is subsequently added, 2,2,2-trifluoroethanol is added dropwise, and copper(I) bromide and the corresponding halogenated benzoic acid of the formula II are added successively or simultaneously to this reaction mixture in any desired sequence,
and acidic work-up is subsequently carried out.

2. Process according to Claim 1, **characterised in that** the reaction is carried out at temperatures between 10° and 80°C.

3. Process according to Claim 1 or 2, **characterised in that** 2,5-bis(2,2,2-trifluoroethoxy)benzoic acid is prepared.

## Revendications

1. Procédé pour la préparation d'acides benzoïques substitués par trifluoroéthoxy de la formule I 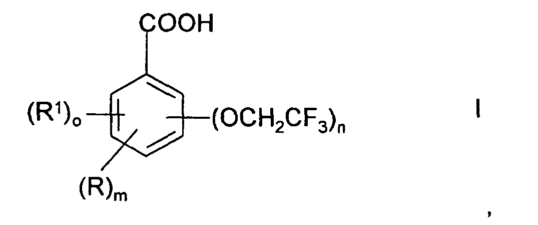
dans laquelle
R, R¹ chacun indépendamment l'un de l'autre, représentent A, OA, COOH, COOA, SA, CF₃, OCF₃, CN, NO₂, Hal, -(CH₂)ₚ-Hal, -O-(CH₂)ₚ-Hal, -S-(CH₂)ₚ-Hal, Ar, -(CH₂)ₚ-Ar, OAr, O(CO)Ar, NH₂, NHA, NA₂, CONH₂, CONHA ou CONA₂,
A représente un radical alkyle comportant de 1 à 4 atomes de C,
Ar représente phényle ou naphthyle, dont chacun est non substitué ou mono-, di- ou trisubstitué par A, OH, OA, CF₃, OCF₃, CN ou NO₂,
Hal représente F, CI, Br ou I,
n représente 1, 2 ou 3,
m, o chacun indépendamment l'un de l'autre, représentent 0, 1 ou 2,
p représente 1 ou 2,
par réaction d'un composé de la formule II 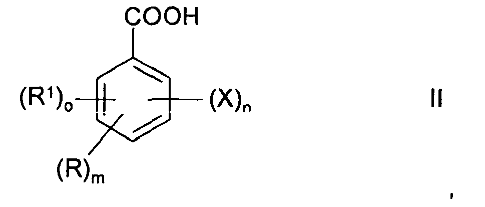
dans laquelle
X représente Cl, Br ou I et
R, R¹, A, Ar, Hal, n, m, o et p présentent la signification indiquée selon la formule I,
où, pour n>1, X peut être identique ou différent,
**caractérisé en ce que**
du tert-butoxyde de potassium et un tris(polyoxaalkyl)amine en tant que catalyseur de transfert de phase sont présentés dans THF, le catalyseur de transfert de phase, lorsqu'il est utilisé, ou la base, s'ils n'ont pas été introduits ensemble, est ensuite ajoutée, du 2,2,2-trifluoroéthanol est ajouté au goutte-à-goutte, et du bromure de cuivre(I) et de l'acide benzoïque correspondant de la formule II sont ajoutés en succession ou simultanément à ce mélange de réaction selon n'importe quelle séquence souhaitée,
et une élaboration acide est ensuite mise en oeuvre.

2. Procédé selon la revendication 1, **caractérisé en ce que** la réaction est mise en oeuvre à des températures entre 10° et 80°C.

3. Procédé selon la revendication 1 ou 2, **caractérisé en ce que** de l'acide 2,5-bis(2,2,2-trifluoroéthoxy)benzoïque est préparé.
